# EUROPEAN PATENT SPECIFICATION

(11) **EP 2 223 106 B1**
(45) Date of publication and mention of the grant of the patent: **19.08.2015**
(21) Application number: 08858117.8
(22) Date of filing: 05.12.2008
(51) Int. Cl.: G01N 33/94

(54) **EMBRYO IMPLANTATION**
IMPLANTATION VON EMBRYOS
IMPLANTATION D'EMBRYON

(30) Priority: 07.12.2007 GB 0723951
(43) Date of publication of application: 01.09.2010
(73) Proprietor: University Of Leicester, Leicester Leicestershire LE1 7RH (GB)
(72) Inventor: KONJE, Justin, Leicester, Leicestershire LE2 7LX (GB)
(74) Representative: Thomson, Craig Richard
(86) International application number: PCT/GB2008/051160
(87) International publication number: WO 2009/071949

(56) References cited:
- WO-A-2005/085873
- WANG HAIBIN ET AL: "Jekyll and hyde: two faces of cannabinoid signaling in male and female fertility." ENDOCRINE REVIEWS AUG 2006, vol. 27, no. 5, August 2006 (2006-08), pages 427-448, XP002515811 ISSN: 0163-769X
- SCHMID P C ET AL: "Changes in anandamide levels in mouse uterus are associated with uterine receptivity for embryo implantation." PROCEEDINGS OF THE NATIONAL ACADEMY OF SCIENCES OF THE UNITED STATES OF AMERICA 15 APR 1997, vol. 94, no. 8, 15 April 1997 (1997-04-15), pages 4188-4192, XP002515813 ISSN: 0027-8424
- TRANGUCH S ET AL: "Molecular complexity in establishing uterine receptivity and implantation" CMLS CELLULAR AND MOLECULAR LIFE SCIENCES, BIRKHÄUSER-VERLAG, BA, vol. 62, no. 17, 1 September 2005 (2005-09-01), pages 1964-1973, XP019200822 ISSN: 1420-9071
- PARIA B C ET AL: "Endocannabinoid signaling in synchronizing embryo development and uterine receptivity for implantation." CHEMISTRY AND PHYSICS OF LIPIDS 31 DEC 2002, vol. 121, no. 1-2, 31 December 2002 (2002-12-31), pages 201-210, XP002515809 ISSN: 0009-3084
- WANG ET AL: "Differential regulation of endocannabinoid synthesis and degradation in the uterus during embryo implantation" PROSTAGLANDINS AND OTHER LIPID MEDIATORS, ELSEVIER, vol. 83, no. 1-2, 26 January 2007 (2007-01-26), pages 62-74, XP005863030 ISSN: 1098-8823
- PARIA B C ET AL: "Fatty-acid amide hydrolase is expressed in the mouse uterus and embryo during the periimplantation period." BIOLOGY OF REPRODUCTION MAY 1999, vol. 60, no. 5, May 1999 (1999-05), pages 1151-1157, XP002516022 ISSN: 0006-3363
- MACCARRONE MAURO ET AL: "Low fatty acid amide hydrolase and high anandamide levels are associated with failure to achieve an ongoing pregnancy after IVF and embryo transfer." MOLECULAR HUMAN REPRODUCTION FEB 2002, vol. 8, no. 2, February 2002 (2002-02), pages 188-195, XP002516023 ISSN: 1360-9947

## Description

### FIELD OF THE INVENTION

The present invention relates to a method for detecting an optimal time for embryo implantation in mammals, particularly humans. Aspects of the invention relate to materials and preparations for use in such methods.

### BACKGROUND OF THE INVENTION

Human reproduction is notorious for being very wasteful. Approximately 50% of all human conceptions are lost. Even more important is the rather low successful fertilisation and pregnancy rate in each menstrual cycle. The cumulative fecundity rate for in normally ovulating women whose partners have a normal sperm function is approximately 70-80% per annum. For those undergoing advanced reproductive techniques (ART) such as in-vitro fertilisation and embryo transfer (IVF-ET) and intracytoplasmic sperm injection (ICSI) and other newer forms of ART the successful pregnancy rates per cycle vary between 10-25%. This is often irrespective of the number of eggs recovered and the quality of the resulting embryos replaced. The variation in replacements (48-72 hours) after egg recovery has also not been demonstrated to significantly affect success rates. This rather highly wasteful process makes ART not only an expensive procedure but an equally stressful one for most couples. Unsuccessful attempts have been made to improve the success rate of ART by the administration of aspirin, progesterone and others chemicals. The failure of any of these to improve successful implantation rates has partly been because of the failure to determine precisely what factors contribute to a successful implantation and early pregnancy maintenance.

Embryo implantation arguably represents the most critical step of the reproductive process in many species. In most animals, the endometrium undergoes a series of changes leading to a period of uterine receptivity called the "window of implantation." Outside of this time, the uterus is resistant to embryo attachment.

The endometrium lines the myometrium and is composed of several functional layers. It functions as a lining for the uterus, preventing adhesions between the opposed walls of the myometrium. During the menstrual cycle or estrous cycle, the endometrium grows to a thick, blood vessel-rich, glandular tissue layer which can receive the embryo.

The process of implantation may be divided into three stages: apposition, adhesion and invasion. During the first stage (blastocyst apposition), trophoblast cells adhere to the receptive endometrial epithelium. This is followed by anchoring of the blastocyst to the endometrial basal lamina and stromal extracellular matrix (ECM) and an embryo-endometrial linkage is established.

Successful implantation requires a receptive endometrium, a normal and functional embryo at the blastocyst developmental stage and a synchronized dialogue between maternal and embryonic tissues. There are a large number of factors that are involved in this process, including adhesion molecules, cytokines, growth factors, lipids and others (for review, see Achache and Revel, Human Reproduction Update, 12(6) 731-746, 2006).

Another factor thought to be involved in normal placentation is the endocannabinoids. Cannabinoids were first isolated as the active ingredients of marijuana and shown to activate mammalian cannabinoid receptors. The endogenous ligands of these receptors are endocannabinoids which are generated from long chain polyunsaturated fatty acid precursors and released into the interstitial space from where they diffuse into the blood stream. The two most studied endocannabinoids are anandamide (AEA) and 2-arachidonyl-glycerol (2-AG) (for review, see Konje et al, Human Reproduction Update, Vol 13, No 5, 501-513, 2007).

Recent animal studies have suggested that AEA is critical for the synchronous development of the blastocyst and endometrium in preparation for successful implantation (Wang H et al Fatty acid amide hydrolase deficiency limits early pregnancy events, J Clin Invest 2006; 116:2122-31).

Plasma AEA levels are regulated by fatty acid amide hydrolase (FAAH), the enzyme that metabolises AEA into arachidonic acid and ethanolamine (Di Marzo et al inactivation of endogenous cannabinoid anandamide in central neurons, Nature 1991; 372: 686-91).

Previous studies have shown a higher plasma AEA level in the follicular phase compared to the luteal phase of the menstrual cycle and a higher expression of FAAH in the peripheral lymphocytes post-ovulation (Habayeb et al, Plasma levels of the endocannabinoid anandamide in women- a potential role in pregnancy maintenance and labour? J Clin Endocrinol Metab 2004; 89: 5482-5487 and Lazzarin, N. et al, Gynecol. Endocrinol 2004; 18, 212-218).

Other studies demonstrated that, in IVF pregnancies, low plasma levels of AEA were associated with pregnancies that progressed beyond the first trimester of pregnancy indicating that a successfully developing conceptus suppresses systemic levels of plasma AEA. That this suppression may be required for normal development of the foetus and pregnancy maintenance is supported by the association of high AEA levels with miscarriages (Maccarrone M et al, Low fatty acid amide hydrolase and high anandamide levels are associated with failure to achieve an ongoing pregnancy after IVF and embryo transfer. MoI Hum Reprod 2002; 8:188-95).

AEA has also been suggested to play a role in miscarriage and preterm labour (EP1 146128 and WO2005/0085873).

Wang et al ("Jekyll and Hyde: Two Faces of Cannabinoid Signalling in Male and Female Fertility", Endocrine Reviews 27(5):427-448), provides a review of what was known in 2006 of the endocannabinoid system. Schmid ("Changes in anandamide levels in mouse uterus are associated with uterine receptivity for embryo implantation"; Proc. Natl. Acad. Sci. USA, vol. 94, pp. 4188-4192, April 1997), provides a study of a link between endocannabinoid levels and early pregnancy failure in mice. Tranguch et al ("Molecular complexity in establishing uterine receptivity and implantation" CMLS,Cell. Mol. Life Sci. 62 (2005) 1964-1973), provides a review of the signalling processes in the endocannabinoid system. Paria et al ("Endocannabinoid signalling in synchronizing embryo development and uterine. receptivity for implantation", Chemistry and Physics of Lipids 121 (2002) 201-210), provides a review of the role of endocannabinoids in mouse embryo development and receptivity. International publication number WO 2005/085873 examines methods of detection of pre-term labour by analysis of endocannabinoids.

None of these studies however investigated the various parts of the menstrual cycle to investigate further changes in plasma AEA and in endometrial FAAH expression during the menstrual cycle and thus determine precisely the optimal time for embryo implantation. Failure of embryo implantation can be one of the causes of infertility. Furthermore, implantation failure is a common occurrence in IVF. Therefore, there is a need to improve embryo implantation by determining more precisely the optimal time for embryo implantation. Failure of embryo implantation can be one of the causes of infertility. Furthermore, implantation failure is a common occurrence in IVF. Therefore, there is a need to improve embryo implantation by determining more precisely the optimal time for embryo implantation and to improve the likelihood for embryo implantation of women undergoing ART.

### SUMMARY OF THE INVENTION

The present invention provides methods for diagnosing/determining the receptivity of the endometrium, thus determining an optimal window for embryo implantation. Specifically, the invention relates to a method for determining the optimal time for implantation of an embryo in a mammalian endometrium comprising detecting the levels of an endocannabinoid during at least three stages of the menstrual cycle in a subject wherein the level of the endocannabinoid in the second phase is higher than in each of the other two phases and wherein a detection of a decrease of the level of the endocannabinoid in the third phase is indicative of the optimal time for embryo implantation. The three stages are early, mid and late stages of the menstrual or estrous cycle.

The invention also relates to methods for determining entry into the luteal phase. Although not part of the claimed invention, also contemplated are methods for improving embryo implantation and for determining ovulation. In a further aspect, the invention relates to methods for predicting a viable pregnancy in a subject mammal undergoing ART. Also described herein, but not part of the claimed invention, is a kit for diagnosing/determining the receptivity of the endometrium.

### BRIEF DESCRIPTION OF THE FIGURES

The aspects of the invention are described with reference to the following drawings which are not considered limiting.
Figure 1: Plasma AEA levels in women, during the menstrual cycle. The menstrual cycle was divided into early follicular d2-7 (n=11), late follicular d8-11 (n=7), ovulatory d12-15 (n=10), early luteal d16-d22 (n=10) and late luteal phase d23-28 (n=9).
Figure 2: IV- ET protocol. This figure shows AEA levels in the three groups of women analysed in the examples. These are healthy, non-treated subjects and women undergoing IVF-ET.
Figure 3: Comparison of plasma AEA, serum E2 and P4 in the pregnant and non-pregnant women during the different stages of IVF/ICSI-ET. The stages are; OR = oocyte retrieval; ET = embryo transfer; PT = pregnancy test; USS = first positive ultrasound scan. AEA = anandamide; E2 = estradiol, P4 = progesterone. Data are presented as mean ± SEM; *P<0.05; **P<0.0001 one-way ANOVA, with Tukey's HSD test.
Figure 4: Plasma AEA levels in pregnant women at 2, 4, 5 and 6 weeks gestation. The data are presented as individual values (n=7) with the mean as the long horizontal bar and the SEM as the shorter horizontal bars. P-values shown above the data were calculated using one-way ANOVA, with Tukey's HSD test.

### DETAILED DESCRIPTION

In a first aspect, the invention relates to a method for determining the optimal time for implantation of an embryo in a mammalian endometrium comprising detecting the levels of an endocannabinoid in the early, mid and late stage of the menstrual cycle in a subject wherein the level of the endocannabinoid in the mid stage of the cycle is higher than in each of the early and late stage and wherein a detection of a decrease of the level of the endocannabinoid in the late stage is indicative of the optimal time for embryo implantation.

The menstrual cycle is divided into different stages or phases. These are the follicular phase, the ovulatory phase and the luteal phase. Preferably, the early stage or phase according to the invention is the follicular stage, the mid stage is the ovulatory stage and the late stage is the luteal phase. The follicular stage occurs at around days 2 to 11, the ovulatory stage at around days 12 to 15 and the luteal stage at around days 16 to 27.

The method may also comprise detecting the levels of AEA in further stages or substages of the menstrual cycle, such as the early follicular stage and late follicular stage. The early follicular stage occurs at around days 2 to 7, the late follicular stage at around days 8 to 11. Thus, according to the invention, the level of endocannabinoid is measured during at least three stages of the menstrual cycle. Furthermore, in one embodiment, the levels of the endocannabinoid are also detected in the early and late luteal phases (around days 16 to 22 and 23 to 27 respectively). Thus, it is possible to obtain a profile of the levels/concentration of AEA during the menstrual cycle. This profile shows the fluctuation of AEA levels during the menstrual cycle and is thus indicative of the optimal window of embryo implantation.

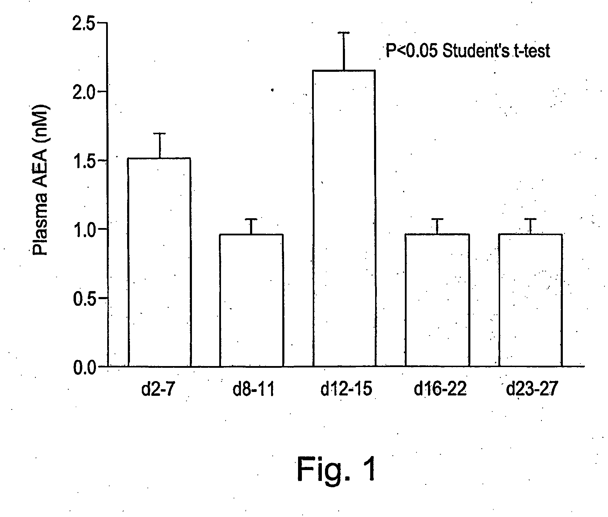

The inventors have surprisingly found that there is a sharp rise in AEA level in the ovulatory phase compared to the foregoing early and late follicular phases. In the ovulatory phase, the level of AEA reaches its peak during the menstrual cycle. After the rise in the ovulatory phase, the AEA level decreases, thus marking the entry into the luteal phase (see figure 1). The inventors have also found that level of AEA is not constant throughout the follicular phase. In the early follicular phase, the level of AEA is high compared to the luteal phase, but lower than in the ovulatory phase, whereas in the late follicular phase, the level of AEA decreases to a level similar to that found in the luteal phase. These findings emphasise the importance of monitoring the level of AEA during the early, mid term and end of the menstrual cycle in order to determined entry into the luteal phase and using AEA as a marker and thus determining an optimal time or window for embryo implantation.

The window of implantation is the time when the endometrium of the uterus is receptive for embryo implantation. This is the optimal time for embryo implantation. This window is marked by the level of AEA and determined according to the methods of the invention. As shown herein, the implantation window is from day 16 to day 27, preferably day 16 to day 20, of the human menstrual cycle.

The invention further relates to a method for determining the optimal time for implantation of an embryo in a mammalian endometrium comprising monitoring the levels of an endocannabinoid during the menstrual cycle of a subject and detecting the peak level of the endocannabinoid during the menstrual cycle wherein the decrease of the level of endocannabioind following the peak is indicative of the optimal time for embryo implantation.

For purposes of the current invention, mammals include, but are not limited to the Order Rodentia, such as mice; Order Logomorpha, such as rabbits; more particularly the Order Carnivora, including Felines (cats) and Canines (dogs); even more particularly the Order Artiodactyla, Bovines (cows) and Suines (pigs); and the Order Perissodactyla, including Equines (horses); and most particularly the Order Primates, Ceboids and Simoids (monkeys) and Anthropoids (humans and apes). The mammals of preferred embodiments are humans.

In a preferred embodiment, the subject is undergoing ART (Assisted Reproductive Technology). Thus, using the methods of the invention, it has become possible to influence the timing of embryo replacement in women undergoing ART.

The methods of the invention involve the detection of an endocannabinoid. The endocannabinoid is preferably anandamide (also known as arachidonylethanolamide or AEA). In alternative embodiments of the invention, the endocannabinoid may be 2-arachidonoylglycerol or palmitoylethanolamide.

The methods of the invention may further comprise the step of taking a sample from the subject mammal for detection. The sample may be taken from blood, plasma, urine, saliva, sputum, cervical or vaginal smears or swabs. The detection may be performed *in vitro*. Preferably, the sample is blood or plasma. In another preferred embodiment, the sample is urine. Detection of the level of the endocannabinoid according to the methods of the invention may be performed in any suitable manner; for example, by means of HPLC, antibodies, receptors, binding molecules, and the like. In certain embodiments, the detection may involve the use of antibodies which bind the endocannabinoid; these antibodies may be labelled with fluorescent or radioactive markers to allow detection, or may themselves be subsequently detected with labelled secondary antibodies or enzymes. Alternatively the antibodies may be used to precipitate the endocannabinoid out of solution. The antibodies may be in solution, or affixed to a solid support. Receptors or binding molecules may be used to detect the endocannabinoid; for example, the receptors CB1 and CB2 are known to bind to AEA, and may be used in detection of AEA.

In certain embodiments of the invention, the endocannabinoid levels may be detected indirectly. For example, it is believed that the enzyme fatty acid amide hydrolase (FAAH) degrades AEA, and that reduced levels of FAAH correlate with elevated levels of AEA. Detection of levels of FAAH may therefore be considered indicative of levels ofAEA.

Although not part of the claimed invention, a method for detecting ovulation is also contemplated in a mammal comprising monitoring the levels of an endocannabinoid during the menstrual cycle of a subject and detecting the peak level of the endocannabinoid during the menstrual cycle.

In another aspect, the invention relates to a method for detecting the entry into the luteal phase in the menstrual cycle in a subject comprising monitoring the levels of an endocannabinoid during the menstrual cycle of a subject and detecting the peak level of the endocannabinoid during the menstrual cycle wherein the decrease of the level of endocannabioind following the peak is indicative of the entry into the luteal phase. Determination of the entry into the luteal phase is important as it is in the luteal phase when successful implantation takes place. Therefore, it is important to distinguish the different stages of the menstrual cycle to allow a more precise determination of the entry into the luteal phase than previously possible.

According to a further aspect of the present invention, there is provided a method for determining embryo implantation in a mammal, comprising detecting the levels of the enzyme fatty acid amide hydrolase (FAAH) in the plasma of a subject mammal during the menstrual cycle, an increase in the level of FAAH during the cycle in the subject mammal being considered indicative of an optimal time for embryo implantation.

Although not part of the present invention, also contemplated is a kit for use in the diagnosis of an optimal time for embryo implantation, the kit comprising a reagent for detection of the levels of an endocannabinoid in a sample. The kit may comprise instructions for use of the reagent. In another embodiment, the reagent comprises antibodies against the endocannabinoid. Furthermore, the kit may comprise means for taking a sample from a subject mammal.

In another aspect, the invention relates to the use of an endocannabinoid in the diagnosis of an optimal time for embryo implantation using the methods as described herein.

In another aspect, the invention relates to the method for improving the likelihood of embryo implantation comprising reducing the levels of an endocannabinoid in a subject The endocannabinoid is preferably AEA. The levels of the endocannabinoid may be reduced by administering antibodies or other binding molecules against AEA; or may be reduced by administering an enzyme that degrades the endocannabinoid, such as FAAH. The levels may also be reduced by administering an activator of an enzyme that degrades the endocannabinoid, such as FAAH. The activator may include pharmacological activators or constitutively active forms of the enzyme. The levels of the endocannabinoid may also be reduced by administering hormones or steroids. More preferably, the levels may be reduced by administering progesterone or human chorionic gonadotrophin, which can up-regulate FAAH activity and subsequently reduce AEA levels. Accordingly, activators of enzymes that degrade endocannabinoids, such as FAAH, include hormones and steroids. Alternatively, the endocannabinoid may be 2-arachidonoylglycerol or palmitoylethanolamide.

Preferably, the subject is undergoing ART, for example IVF or ICSI. For example, the subject may have abnormal levels of AEA compared to a reference level of AEA in a normal and healthy subject which reduce the likelihood for embryo implantation. The reference level may be based on typical levels at six to eight weeks of gestation. The reference level is preferably equal to or less than 2.OnM plasma AEA. As shown in the examples, the mean level of AEA in the women who had a successful ART were significantly lower than at egg retrieval. Using the methods of the invention, the likelihood of embryo implantation can be improved, in particular in women who suffer from infertility and who are unlikely to implant an embryo. Thus, a window of implantation can be induced according to the methods of the invention and the methods of the invention provide the opportunity to influence the timing of embryo replacement in women undergoing ART. The methods of the invention can thus be used to treat subjects who suffer from infertility. For example, AEA levels may be reduced to enhance the likelihood of successful implantation.

The inventors have also found that a significant reduction in AEA levels from the day of oocyte retrieval to the day of embryo transfer in individuals undergoing ART correlated with a viable pregnancy. In comparison, there was no significant difference in the AEA. levels between oocyte retrieval and embryo transfer in individuals undergoing ART who were unsuccessful in getting pregnant. Accordingly, another aspect of this invention relates to a method for predicting the likelihood of a viable pregnancy comprising measuring the levels of AEA on the day of oocyte retrieval and embryo transfer, wherein a reduction in the levels of AEA, from oocyte retrieval to embryo transfer indicates successful implantation and an increased likelihood of a viable pregnancy. The reduction may be at least 10 %, or more preferably 40 % or more.

Reduced levels of AEA associated with implantation in the mouse are believed to be regulated by sex steroid hormones, such as estradiol and progesterone. The inventors have also found that a similar association exists in humans. Furthermore, the inventors have found that the levels of estradiol (E2) fall from the day of oocyte retrieval to embryo transfer and increase from embryo transfer to the day of the pregnancy test and the ultrasound scan in pregnant women who have undergone ART. In comparison, the inventors have found a significant reduction in the levels of E2 from the day of oocyte retrieval and embryo transfer to the day of the pregnancy test in individuals who have undergone ART and were unsuccessful in becoming pregnant. Accordingly, a significant reduction in E2 levels from ooctye retrieval or embryo transfer to the day of the pregnancy test is predictive of unsuccessful embryo implantation and thus, an unviable pregnancy. The methods of the invention therefore also relate to measuring the levels of E2 at the time of oocyte retrieval and/or embryo transfer and at the pregnancy test, wherein a decrease in E2 levels at the time of the pregnancy test from the oocyte retrieval and/or embryo transfer indicates unsuccessful embryo implantation.

In addition to the above, the inventors have also found that levels of endocannabinoids, such as AEA, post implantation can be used to determine the likelihood of a viable pregnancy. The levels of AEA in pregnant individuals following ART were found to increase from a low level at embryo transfer to a higher level at 4 or 5 weeks gestation. This high level of AEA dropped again to a lower level at 6 weeks gestation. The rise in AEA levels from embryo transfer to 4 or 5 weeks gestation were found to be required for a viable pregnancy. Accordingly, the methods of this invention also relate to measuring the levels of AEA at embryo transfer and at 4, 5 and 6 weeks gestation, wherein a rise in the levels of AEA from embryo transfer to 4 or 5 weeks gestation and/or a drop in the levels of AEA from 4 or 5 weeks gestation to 6 weeks gestation indicates successful implantation of the embryo and a viable pregnancy.

The invention is further described by reference to the following non limiting examples.

### Example 1

The following examples show quantitation of the levels of anandamide (AEA) during the menstrual cycle to determine whether it changes during a normal menstrual cycle, and quantitation of the levels of anandamide (AEA) to determine levels in women undergoing ART at the time of oocyte recovery, at the time of embryo replacement and serially thereafter to investigate whether the success of the ART could be determined by levels of AEA.

The study was a cross-sectional study. Plasma AEA levels were measured using a sensitive high performance liquid chromatography-mass spectrometry HPLC MS/MS method. Blood samples were taken from 47 women with a median age of 34 years (range 20-45) and BMI 22kg/ni2 (range 19-27) with regular menstrual cycles, with no medical problems and not on any medication for the preceding 6 months. The menstrual cycle was divided into early follicular d2-7 (n=11), late follicular d8-11 (n=7), ovulatory d12-15 (n=10), early luteal d16-d22 (n=10) and late luteal phase d23-28 (n=9). Uterine biopsies were taken from hysterectomy specimens taken for benign conditions and subjected to immunohistochemistry for fatty acid amide hydrolase with polyclonal antibodies. Samples were also taken from 36 women undergoing either ICSI or IVF-ET. AEA was measured using a sensitive HPLC MS/MS method. For those undergoing ART plasma was collected at the time of egg recovery, at the time of embryo transfer, first and second biochemical pregnancy tests and ultrasound scan for confirmation of fetal viability.

The results demonstrate remarkable changes in the levels of AEA during the menstrual cycle. AEA levels were significantly higher around ovulation in comparison to either the pre-ovulatory phases or post-ovulatory phases as shown in Figure 1. FAAH expression in the endometrial stroma was unchanged throughout the early, mid and late follicular phases but increased during the mid to late luteal phase reaching a maximum in the late luteal phase.

AEA levels varied during the menstrual cycle with the highest values around the time of ovulation. These changes were similar to two values we published for the follicular and luteal phases. The difference between our present data and previous data is that we broke up the menstrual cycle into days and were able to measure values at the time of ovulation.

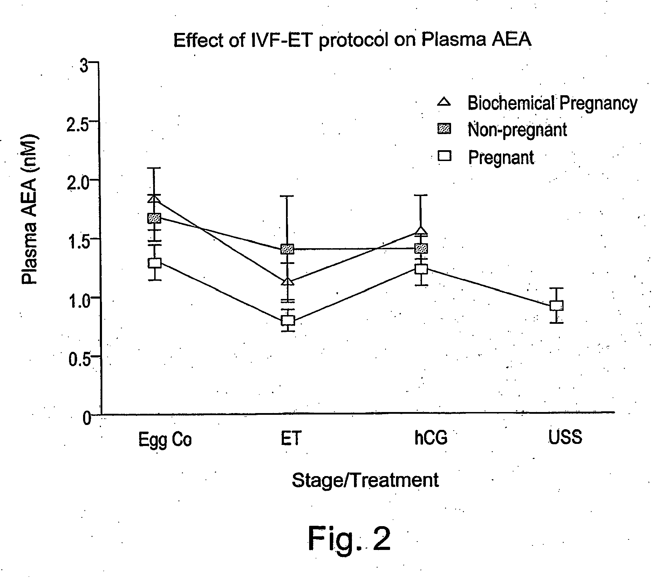

Samples from 36 women undergoing ART were analyzed for AEA levels. Six of these had a pregnancy confirmed by a fetal heart on ultrasound scan, 3 were biochemical pregnancies and the rest were unsuccessful. Figure 2 shows the changes in AEA levels in the three groups of women used in the experiment. The mean AEA levels in the women who had a successful ART were significantly lower than that at egg retrieval (P<0.001) while that in those who had a biochemical pregnancy fell but not significantly. The values in those who did not have a successful pregnancy did not change and indeed in some of them the levels rose.

### Example 2

The following example was a case-control prospective study of women undergoing ovarian stimulation for infertility in an in-vitro fertilisation or intracytoplasmic sperm injection with embryo transfer (IVF/ICSI-ET) programme. The cases were those who had a positive pregnancy test (β-human chorionic gonadotrophin (β-hGG) >5IU/L) and ultrasound scan evidence of a viable intrauterine pregnancy and the controls were those who had a negative pregnancy test (β-hCG <5IU/L).

All the women studied were healthy (i.e. had no other medical disorders) and were not on any long-term medications. None of them smoked cigarettes or admitted to using any recreational drugs. The women were enrolled after written signed informed consent was obtained. The study was approved by the Leicestershire and Rutland Local Research Ethics Committee (i.e. Institutional Review Board).

Ovarian stimulation by a long protocol using gonadotrophin hormone (GnRH) agonist (Supercur; Aventis Pharma Ltd, Kent, UK) was used to down regulate the pituitary. This was commenced in the mid luteal phase of the previous cycle and was administered as a daily subcutaneously injection until ovulatory hCG was given. Stimulation with subcutaneous gonadotrophins was initiated once sonographic evidence of no ovarian follicular activity and serum estradiol levels were below 200pmol/L. The gonadotrophin was either human menopausal gonadotrophin (hMG) (Menopur; Ferring, Langley, UK) or recombinant follicle stimulating hormone (rFSH) (Puregon; Organon Laboratories Ltd, Cambridge, UK) and the dosage was based on the patient's age, BMI. and early follicular phase serum FSH level. Follicular monitoring was performed by serial transvaginal ultrasound scan and serum estradiol (E2) measurements (every 2-3 days) (22). The subcutaneous hCG injection (10,000IU; Pregnyl; Organon Laboratories Ltd, Cambridge, UK) was administered to induce final oocyte maturation when there were at least 4 follicles measuring more ≥17mm in diameter and the endometrial thickness was at least 8mm. Oocyte retrieval was performed 36 hours later by transvaginal ultrasound guided aspiration of the ovarian follicles.

Standard IVF/ICSI-ET procedures were followed (Brauda P & Rowell P, Assisted conception. II -In vitro fertilisation and intracytoplasmic sperm injection. British Medical Journal 2003;327:852-855). Fertilisation was assessed 16 hours later and the two best quality embryos for each woman (identified by an embryologist using various morphological features) were transferred into the uterus of each patient 48 hours after oocyte retrieval.

Progesterone (P4) vaginal pessaries (400mg twice daily; Cyclogest, Shire Pharmaceuticals, Basingstoke, Hampshire, UK) were commenced after transfer of embryos to support the luteal phase until the day of pregnancy test for the non-pregnant group (control) and up to 12 weeks for the pregnant group (cases)(Nosarka S, et al, Luteal phase support in in vitro fertilization: Meta-analysis of randomized trials, Gynaecologic and Obstetric Investigation 2005; 60(2):67-74). Urinary pregnancy test and serum β-hCG measurements were performed two weeks following embryo transfer. Women with a serum β-hCG level <5 IU/L were considered not pregnant; those with a β-hCG of >5IU/L had a second serum β-hCG a week later and if the second β-hCG value was >1500 IU/L. a transvaginal ultrasound scan was performed 4 weeks after embryo transfer (i.e. equivalent to 6 weeks gestation). The women were asked to report any vaginal bleeding or significant abdominal pain. The non-pregnant women reported vaginal bleeding which started one or two days before the urinary β-hCG pregnancy test.

At oocyte collection, embryo transfer, urinary pregnancy tests in cases and controls and at ultrasound scan, 8ml of blood were collected from each woman in both groups for AEA and hormonal (E2, P4, β-hCG) measurements. Samples were taken approximately 30 minutes before oocyte retrieval, 30 minutes before embryo transfer, on the day of urinary pregnancy test (4 weeks) and from the pregnant women on the day of a positive ultrasound scan (6 weeks). In a subset of seven pregnant women extra samples for the measurement of plasma AEA were collected at week 5 of pregnancy.

### Plasma AEA measurement

Plasma AEA concentrations were estimated using our validated Ultra Performance Liquid Chromatography-Mass Spectrometry (UPLC-MS/MS) method with lipid extraction and quantification completed within 2 hours of blood sample collection (Lam PC et al, Ultra performance liquid chromatography tandem mass spectrometry method for the measurement of anandamide in human plasma, Analytical Biochemistry 2008; 380(2):195-201).

### Hormone measurements

Serum estradiol (E2), progesterone (P4) and β-human chorionic gonadotrophin (β-hCG) measurements were performed on an automated ADVIA Centaur Assay System (Bayer Diagnostics, Newbury, and Berkshire, UK). The inter-assay coefficients of variation for E2, P4, β-hCG were 5.6, 3.6, and 2.9%, respectively, whilst the intra-assay coefficients of variation were 8.4, 5.2 and 2.7%, respectively.

### Statistics

A power calculation based on previous published methods for the measurement of plasma AEA(20, 21) indicated that a minimum of 6 women in the pregnant and non-pregnant groups would allow a clinically significant difference of 40% in plasma AEA concentrations to be observed with 80% power, assuming 2-sided α= 0.05. Statistical analysis of the data was performed using one-way ANOVA with Tukey's honestly significance difference test and comparison between groups was performed using the Student's unpaired t-test; a P<0.05 was considered significant. Data relating to patient demographics and cycle characteristics are presented as mean ± SD (with the range) or as median where appropriate. Plasma AEA and serum E2, P4 and β-hCG measurements are presented as mean ± SEM. Linear regression analysis was used to determine the associations between plasma AEA and serum E2, P4, β-hCG. Data were analyzed using InStat Version 3.01 (GraphPad Software Inc, San Diego, California, USA; www.graphpad.com).

### Results

### Subjects and cycle characteristics of the study groups

In total, 24 infertile women were recruited into the study; 12 pregnant (pregnant group) and 12 non-pregnant (non-pregnant group). They were matched for age, BMI, duration of infertility, type of infertility (primary; secondary), cause of infertility, basal FSH and LH levels as shown in Table 1.

These women were from a cohort of 78 women undergoing IVF/ICSI-ET. Once a pregnant case was identified the next non-pregnant case who was matched for age, BMI, duration, type and cause of infertility, was selected as control. The treatment protocols in the two groups of women were very similar (Table 2). There was, however, a significantly higher number of ovarian stimulation days for the non-pregnant when compared to the pregnant group (P=0.01). Despite the fertilisation rates not being significantly different in both groups (P=0.11), the pregnant women produced almost double the number of embryos for transfer compared to the non-pregnant women (P=0.01). In addition, the total number of good quality embryos available on the day of embryo transfer was significantly higher in the pregnant group (P=0.003).

### Plasma AEA levels

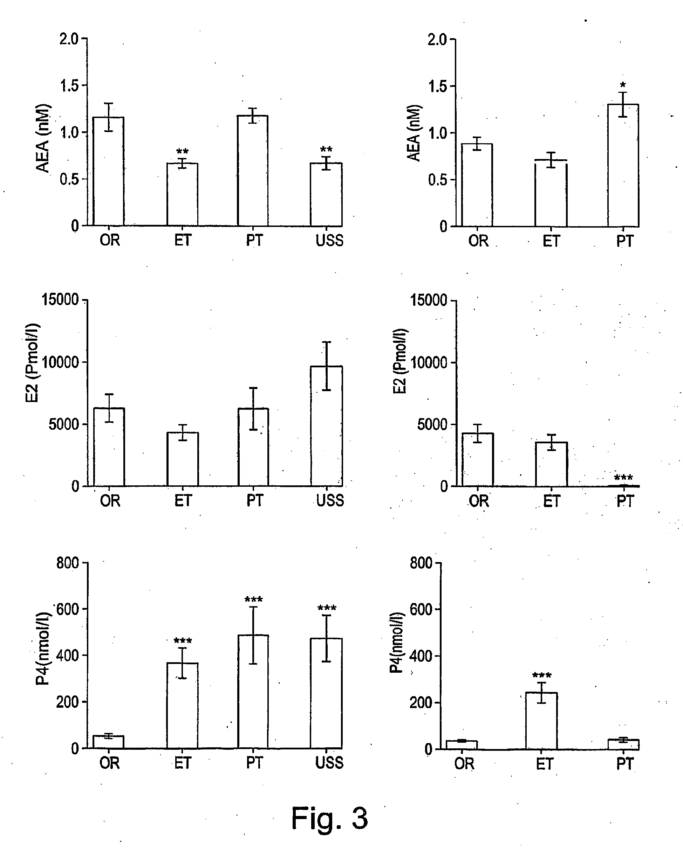

In pregnant women, the highest plasma AEA levels (mean ± SEM) were observed at the time of oocyte retrieval (1.16 ± 0.15nM) and pregnancy test (1.18 ± 0.09nM). The lowest measurable plasma AEA levels were from samples taken just prior to embryo transfer and on the day of a positive ultrasound scan (0.66 ± 0.04nM) and (0.67 ± 0 .06nM), respectively (Table 3). In the pregnant group, plasma AEA levels decreased significantly (P<0.001) from the day of oocyte retrieval to the day of embryo transfer with a mean fall of 43.2%. However, AEA levels rose significantly (P<0.001) from the day of embryo transfer to that of pregnancy test followed by another significant (P<0.001) decrease from the time of pregnancy test to the first ultrasound scan (Figure 3).

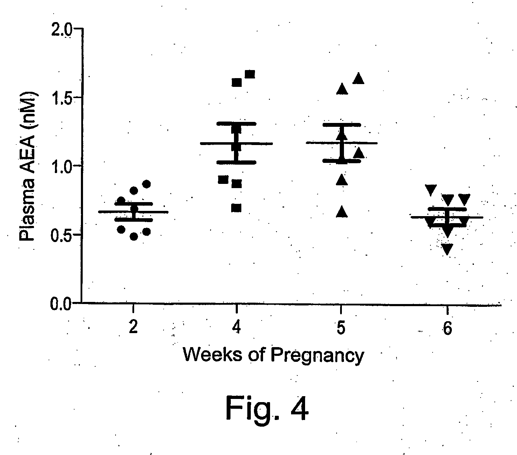

The changes in plasma AEA levels from embryo transfer to 6 weeks gestation (4 weeks after embryo transfer) in the 7 pregnant women who had an additional blood sample taken at 5 weeks are shown in Table 4 and Figure 4. There was a significant (P=0.006) increase in plasma AEA levels from the embryo transfer day (i.e. week 2 gestation) (0.67 ± 0.06nM) to that (1.17 ± 0.14nM) at week 4 gestation. There was no significant (P=0.97) change in plasma AEA levels at week 4 to that at week 5 (1.18 ± 0.13nM), but there was a significant decrease (P<0.05) from week 4 (1.17 ± 0.14nM) to week 6 (0.66 ± 0.06nM). A similar significant decrease was seen from the levels at 5 weeks to that at 6 weeks gestation (P=0.003).

By contrast, in the non-pregnant group the highest plasma AEA levels (1.31 ± 0.14nM) were seen on the day of the pregnancy test, and the lowest levels were on the day of oocyte retrieval (0.89 ± 0.07nM) and on the day of embryo transfer (0.71 ± 0.08nM). Unlike the significant changes seen in plasma levels of the pregnant women from the oocyte retrieval to the day of embryo transfer, there was no statistically significant difference in plasma AEA levels between oocyte retrieval and embryo transfer in non-pregnant group. The mean difference in the decline in plasma AEA levels between the two periods was only 20.2%. There was a significant increase in plasma AEA levels from the day of oocyte retrieval to the day of pregnancy test (P<0.05) and a significant increase from the day of embryo transfer to the day of pregnancy test (P<0.0001 ).. An analysis of plasma AEA levels between the pregnant and non-pregnant groups at the 3 studied time periods showed that there was no statistically significant difference between the two groups of women (Table 3).

### Serum E2, P4, and β-hCG levels

The highest level (9689 ± 1935pmol/L) of serum E2 in the pregnant women was on the day of ultrasound scan and the lowest (4324 ± 635pmol/L) on the day of embryo transfer (Figure 3). Although there appeared to be a subtle fluctuation in the serum E2 concentrations over the course of treatment, these data were not significantly different. For the non-pregnant group, the highest level (4295 ± 622.8pmol/L) of serum E2 was noted on the day of oocyte retrieval and the lowest level (129.6 ± 21.7pmol/L) on the day of the negative pregnancy test (Figure 3). The decrease in serum E2 concentrations observed from the day of oocyte retrieval to the day of pregnancy test in the non-pregnant group was significantly different (P<0.0001). A similar decrease in serum E2 concentrations from 3534 ± 616.5pmol/L on the day of embryo transfer to 129.6 ± 21.7pmol/L on the day of pregnancy test was also significantly different (P<0.0001). When the serum E2 levels in the pregnant and non-pregnant groups were compared (Table 3) only the levels on the day of pregnancy test were significantly different (P=0.002).

Serum P4 levels in the pregnant group were highest (483.7 ± 123nmol/L) on the day of positive pregnancy test and on the day of ultrasound scan (471.7 ± 99.3nmol/L) (Figure 3). The P4 level was lowest (49.6 ± 11.2nmol/L) on the day of oocyte retrieval and increased significantly (P<0.0001) on the day of embryo transfer (364.9 ± 64.1nmol/L). The serum P4 levels on the day of embryo transfer, pregnancy test and ultrasound scan were not significantly different (Figure 3). In the non-pregnant group, however, the highest P4 level (241.8 ± 39.4nmol/L) was on the day of embryo transfer and the lowest (32.2 ± 4.8nmol/l) was on the day of oocyte retrieval. There was a significant (P<0.0001) increase in serum P4 levels from that on the day of oocyte retrieval to that on the day of embryo transfer, and a significant decrease to that (42.1 ± 8.5nmol/L) on the day of negative pregnancy test (P<0.0001). A comparison between the serum P4. levels in the pregnant and non-pregnant groups (Table 4) showed that only the levels found on the day of pregnancy test were significantly different (P=0.002).

Serum β-hCG levels measured exactly two weeks after embryo transfer were significantly higher in the pregnant group (202.5 ± 29.59 IU/L; range 70-392 IU/L) than in the non-pregnant group (all were <2 IU/L).

### Hormonal associations

The possible relationships between plasma AEA levels and serum levels of the sex steroids (E2, P4) in the pregnant and non-pregnant groups at oocyte retrieval, embryo transfer and pregnancy test were examined. There was no significant correlation in the pregnant and non-pregnant groups between plasma AEA levels and serum P4 levels (P=0.95 and P=0.15), respectively. While there was no statistically significant correlation between plasma AEA levels and E2 levels in the pregnant group (P=0.95) the relationship in the non-pregnant group was statistically significant (P=0.022). Analyzing the data at positive ultrasound scan (6 weeks) separately showed that there were no statistically significant correlations between plasma AEA levels and serum E2 levels (P=0.88), plasma AEA levels and serum P4 levels (P=0.75), and plasma AEA levels and serum β-hCG levels (P=0.58).

### Discussion

Embryo quality (Bergh PA & Novat, The impact of embryonic development and endometrial maturity on the timing of implantation, Fertility & Sterility 1992; 58(3):537-542) and an appropriately hormonally-primed receptive endometrium (Norwitz ER et al, Implantation and the survival of early pregnancy, New England Journal of Medicine 2001; 345(19):1400-1408 and Diedrich K, et al, The role of the endometrium, and embryo in human implantation, Human Reproduction 2007; Update 13(4):365-377) are considered to be important factors for successful implantation. A third recognised ingredient in successful implantation is successful cross-talk between the embryo and the primed receptive endometrium (Dey SK et al, 2004 Molecular cues to implantation, Endocrine Reviews 2004;25(3):341-373 and Paria BC, et al, Deciphering the cross talk of implantation: advances and challenges, Science 2002; 296(5576):2185-2188). A current lack of understanding of the molecules that regulate this process (Dimitriadis E, et al, Cytokines, chemokines and growth factors in endometrium related to implantation, Human Reproduction Update 2005;11(6):613-630), means implantation failure remains a major obstacle to improving pregnancy outcomes following fertilisation.

It can be implied from animal studies demonstrating that AEA is involved in implantation (Das SK, et al, Cannabinoid ligand-receptor signalling in the mouse uterus. The Proceeding of National Academy of Sciences 1995;USA 92(10): 53.7-542) that a similar process occurs in women. Examining women during their treatment in an IVF-ET programme provided an opportunity to accurately study the relationship between plasma AEA and the steroids E2 and P4 at the time of ovulation, the implantation window and early pregnancy. In addition, it allowed for the comparison of plasma AEA. levels in a cohort of women who had a positive implantation as evidenced by a viable pregnancy at 6 weeks gestation and another cohort who failed to implant.

An important finding in this study was the statistically significant reduction (43%) in plasma AEA levels in the pregnant group, but not in the non-pregnant group, that occurred during the transition from the day of oocyte retrieval to the day of embryo transfer. If the plasma AEA levels reflect those in the uterus, then this observation would be in keeping with the findings of Guo et al. that the levels of AEA in the uterine cavity fall to enable successful implantation (Guo Y, et al, N-Acylphosphatidylethananolamine-hydrolyzing phospholipase D is an important determinant of uterine anandamide levels during implantation. Journal of Biological Chemistry 2005; 280(25):23429-23432).

The changes in plasma AEA levels from the day of embryo transfer to 6 weeks gestation revealed that in a viable pregnancy, a low AEA level was required on the day of embryo transfer and higher levels at 4 and 5 weeks gestation, only significantly falling thereafter. AEA levels at the 6^{th} week of pregnancy were similar to the previously reported levels in early pregnancy (Maccarrone M, et al, Relationship between decreased anandamide hydrolase concentrations in human lymphocytes and miscarriage, Lancet 2000; 355(9212):1326-1329 and Habayeb OM, et al, Plasma anandamide concentration and pregnancy outcome in women with threatened miscarriage, JAMA 2008; 299(10):11135-1136) and none of our pregnant women had AEA values >2nM at 6 weeks and so none had miscarried by 12 weeks, a finding in keeping with our previous observations (Habayeb et al).

Our findings suggest that plasma AEA levels fluctuate between ovulation and very early pregnancy and that for successful pregnancy to occur a higher plasma AEA level is probably required at the time of ovulation and a significantly lower level at the beginning of implantation to assist in uterine receptivity. At the start of implantation an interaction between the embryo and endometrium occurs and higher AEA levels are probably then required or the levels increase due to the development of the trophoblast.

With regards to the regulation of plasma AEA, our data and the well recognised physiological levels of hormones controlling the periods studied (Dodson KS, et al, Plasma sex steroid and gonadotrophin patterns in human menstrual cycles, British Journal of Obstetrics and Gynaecology 1975; 82:602-614) provide clues to possible mechanisms regulating AEA levels. We observed no statistically significant relationship between plasma AEA and P4 at implantation and during early pregnancy suggesting that there was no significant association between plasma AEA and P4. Previous studies have suggested that progesterone down regulates AEA levels during the implantation window. Since both the pregnant and non-pregnant groups in this study had similar high levels of progesterone on the day of embryo transfer prior to starting the extra progesterone supplement to support their luteal phase, similar low plasma AEA levels in both groups would have been expected. The data, however, showed that the pregnant group had high P4 and high AEA levels, and the non-pregnant group had low P4 levels , and high plasma AEA levels suggesting that P4 is not the controlling factor in these two groups of women. Furthermore, the lack of correlation between the plasma AEA levels and serum P4 at 6 weeks gestation also supported this conclusion. These data are qualitatively similar to our previous study of normal cycling women (EI-Talatini MR, et al, The relationship between plasma levels of the endocannabinoid, anandamide, sex steroids and gonadotrophins during the menstrual cycle, Fertility and Sterility 2009 (in press) where no association between AEA and P4 was found, Thus we can conclude that AEA in women is most probably not regulated by P4.

Since oocyte retrieval can be assumed to correspond to the ovulation stage in the natural cycle, embryo transfer equivalent to the implantation window or mid luteal phase of the natural cycle and a negative pregnancy test is equivalent to the early follicular phase of the next cycle (these women started bleeding due to the withdrawal of their progesterone levels), it is reasonable to conclude that the changes in plasma AEA levels correlate with well-documented physiological changes in gonadotrophin levels (Duncan WC et al, Luteinizinig hormone receptor in the human corpus luteum: lack of down-regulation during maternal recognition of pregnancy, Human Reproduction 1996; 11(10):2291-2297) and that there is an inverse relationship with E2. It is well known that hCG can mimic the action of LH (Speroff L & Fritz MA ,The Endocrinology of Pregnancy, In: Speroff L ed. Clinical Gynecologic Endocrinology and Infertility 7th ed. Lippincott, Williams & Wilkins Philadelphia: 2005 USA; pp274-278) in the corpus luteum. Indeed, hCG injection is given to all these women 36 hours before oocyte retrieval to induce ovulation (Fischer RA et al, Ovulation after intravenous and intramuscular human chorionic gonadotropin, Fertility and Sterility 1993; 60(3):418-422), mimicking the LH surge. However, in pregnant women, at the time of a positive pregnancy test, physiologically, LH levels and hCG levels are elevated (Duncan et al).

The hCG secreted from the syncytiotrophoblast one day after implantation and the LH secreted from maternal anterior pituitary both support the corpus luteum in its role of producing steroids which are important for pregnancy survival, leading to elevated P4 and E2 levels. The corpus luteum is totally dependent on hCG levels until the seventh week of pregnancy (Baird DD et al, Rescue of the corpus luteum in human pregnancy, Biology of Reproduction 2003; 68(2):448-456) and from then to the 10^{th} week of pregnancy the function of the corpus luteum is gradually replaced by placenta (Speroff et al). Since we have concluded that AEA may be regulated by gonadotrophins in non-pregnant women, it is not surprising that AEA levels in pregnant women were also elevated at 4 and 5 weeks gestation as LH levels are elevated. However, at the 6^{th} week of pregnancy, even though hCG levels remain high, the LH receptors in the corpus luteum are gradually declining and the corpus luteum is starting to regress as the placenta begins to produce all the hormones that support the pregnancy (Speroff et al). Subsequently, LH levels becomes very low and the levels of AEA accordingly decline supporting the observation that plasma AEA levels decline further after the 6^{th} week of gestation.

The fact that we found no relationship between plasma AEA and serum β-hCG, which is exclusively produced from trophoblast, is in keeping with the findings of Maccarone et al., suggesting that AEA during the early weeks of pregnancy is mainly regulated by maternal LH (Maccarrone et al, 2002).

All of these data are consistent with the notion that during the follicular phase of the menstrual cycle FSH is the main controlling factor regulating plasma AEA levels, whereas during ovulation, implantation and early pregnancy, AEA is regulated by LH and E2.

In summary, our results suggest that in a viable pregnancy AEA levels fluctuate from the time of ovulation, to early pregnancy, the highest levels being at the time of ovulation and the lowest at 6 weeks gestation. A significant decline in the plasma AEA levels (around 40%) at the implantation window is important. Additionally, the changes in plasma AEA levels from oocyte retrieval to embryo replacement (implantation window) could be use to predict the cases in which implantation will be successful in women undergoing IVF/ICSI-ET.

**Table 1. Characteristics of the study group**

| BMI = body mass index; IVF = in-vitro fertilization; ICSI = intercytoplasmic sperm | | | |
|---|---|---|---|
| | **Pregnant (n=12)** | **Non- Pregnant (n=12)** | **P-value** |
| **Age (years)** | 32.17 ± 3.27 (28-38) | 32.75 ± 3.72 (27-38) | 0.69 |
| **BMI (Kg/m²)** | 23.42 ± 3.60 (19-30) | 23.83 ± 3.12 (19-29.5) | 0.76 |
| **Duration of infertility (years)** | 4.66 ± 1.72 (2-7) | 4.17 ± 1.80 (2-7) | 0.49 |
| **Type of infertility:** | 7 | 7 | |
| **Primary infertility** | | | |
| **Secondary infertility** | 5 | 5 | |
| **Cause of infertility:** | | | |
| **Male** | | 3 | |
| **Tubal** | 3 | 3 | |
| **Unexplained** | 5 | 6 | |
| **Treatment** | | | |
| **IVF-ET** | 5 | 6 | |
| **JCSI-ET** | 7 | 6 | |
| **Basal hormones:** | | | |
| **FSH (IU/l)** | 5.79 ± 1.89 (3.3-10) | 6.19 ± 1.74 (4-9.60) | 0.59 |
| **LH (IU/l)** | 4.55 ± 1.78 (3.0-8.4) | 4.31 ± 1.77 (2.3-7.7) | 0.75 |

| | | | |
|---|---|---|---|
| injection; FSH = follicle stimulating hormone; LH = luteinizing hormone. | | | |

**Table 2. Details of the dosage and treatments offered to the studied groups and the results of the ovulation stimulation**

| rFSH = recombinant follicle stimulating hormone; hMG = human menopausal | | | | | |
|---|---|---|---|---|---|
| | **Pregnant (n=12)** | | **Non-Pregnant (n=12)** | | **P-value** |
| | Mean ± SD (range) | Median | Mean ± SD (range) | Median | |
| **Days of stimulation** | 10.75 ± 1.76 | 11.5 | 12.17 ± 0.71 | 12 | **0.01** |
| | (7-12) | | (11-14) | | |
| **Dose of Puregon (rFSH) IU/L** | 1639 ± 496.6 (1000 -2475) | 1800 | 2463 ± 1546 (1350-5400) | 1988 | 0.21 |
| **Dose of Menopur (hMG) IU/L** | 4275 ± 2679 (1650-8250) | 3150 | 4004 ± 2551 (1800-9150) | 3900 | 0.86 |
| **Fertilisation rate (2PN)** | 59.86 ± 20.72 (13.3-100) | 63.95 | 45.06 ± 22.67 (22.2-100) | 38.75 | 0.11 |
| **Total number of embryos per women available on the day of embryo transfer** | 6.82 ± 3.99 (2-15) | 7 | 3.42 ± 1.8 (2-8) | 2.5 | **0.01** |
| **Total number of good embryos per woman available on the day of embryo transfer** | 5.57 ± 3.31 (2-11) | 5.5 | 2.33 ± 1.07 (1-4) | 2 | **0.003** |

| | | | | | |
|---|---|---|---|---|---|
| gonadotrophins; 2PN = Two pronuclei. Data are expressed in mean ± SD, median and range. P-values were calculated using Student's unpaired *t*-test. | | | | | |

**Table 3.Serum progesterone (P4) estradiol (E2) and plasma anandamide (AEA) levels in the pregnant and non pregnant groups during IVF/ICSI treatment stages.**

| | **P4 Pregnant** | **P4 Non Pregnant** | **P** | **E2 Pregnant** | **E2 Non Pregnant** | **P** | **AEA Pregnant** | **AEA Non Pregnant** | **P** |
|---|---|---|---|---|---|---|---|---|---|
| **Oocyte Retrieval** | 49.63 ± 11.24 | 32.25 ± 4.76 | 0.17 | 6317 ± 1111 | 4295 ± 662.8 | 0.132 | 1.16 ± 0.15 | 0.89 ± 0.07 | 0.11 |
| **Embryo Transfer** | 364.9 ± 4.08 | 241.8 ± 39.36 | 0.11 | 4324 ± 635.4 | 3534 ± 616.5 | 0.382 | 0.66 ± 0.04 | 0.71 ± 0.08 | 0.62 |
| **Pregnancy Test** | 483.7 ± 123 | 42.08 ± 8.5 | **0.002** | 6238 ± 1695 | 129.6 ± 21.71 | **0.002** | 1.18 ± 0.09 | 1.31 ± 0.14 | 0.44 |
| **Ultrasound Scan** | 471.7 ± 99.27 | | | 9689 ± 1935 | | | 0.67 ± 0.06 | | |

| | | | | | | | | | |
|---|---|---|---|---|---|---|---|---|---|
| Data are expressed as mean ± SEM. P-values were calculated using Student's unpaired *t*-test. | | | | | | | | | |

**Table 4. Plasma AEA concentrations in pregnant women from samples taken 30 minutes prior to embryo transfer to the time of ultrasound scan.**

| **Patient number** | **AEA-ET (nM)** | **β-hCG1 (IU/L)** | **AEA-1 (nM)** | **β-hCG2 (IU/L)** | **AEA-2 (nM)** | **USS (Weeks)** | **AEA-USS (nM)** |
|---|---|---|---|---|---|---|---|
| **1** | 0.571 | 132 | 0.979 | 2310 | nd | 6 | 0.424 |
| **2** | 0.871 | 299 | 1.279 | 4800 | 0.684 | 6 | 0.764 |
| **3** | 0.529 | 70 | 1.151 | 4141 | 1.115 | 6⁺⁴ | 0.533 |
| **4** | 0.694 | 120 | 1.619 | 4805 | 1.660 | 6⁺³ | 0.613 |
| **5** | 0.865 | 392 | 1.465 | 4950 | nd | 6 | 0.652 |
| **6** | 0.373 | 256 | 1.286 | 8968 | nd | 6⁺⁶ | 0.556 |
| **7** | 0.640 | 303 | 1.231 | 6654 | nd | 6⁺³ | 0.794 |
| **8** | 0.539 | 123 | 0.881 | 4000 | 1.245 | 6⁺⁵ | 0.408 |
| **9** | 0.489 | 296 | 0.700 | 4430 | 1.074 | 6⁺⁴ | 0.770 |
| **10** | 0.674 | 161 | 0.933 | 5978 | nd | 6 | 1.234 |
| **11** | 0.823 | 179 | 1.676 | 2618 | 0.914 | 6⁺⁴ | 0.837 |
| **12** | 0.751 | 99 | 0.911 | 3000 | 1.583 | 6 | 0.604 |

| | | | | | | | |
|---|---|---|---|---|---|---|---|
| The table shows plasma AEA measurements at embryo transfer (AEA-ET), at 4 weeks (AEA-1) at 5 weeks (AEA-2) and at 6 weeks (AEA-USS). Serum β-hCG at 4 weeks (β-hCG 1) and 5 weeks (β-hCG2) gestation are shown. USS = the date of transvaginal scan (weeks^{+days}). AEA = AEA concentration; β-hCG = serum β-hCG cocnentrations; nd = not determined because β-hCG1 was sufficiently high enough to indicate the pregnancy was progressing. | | | | | | | |

## Claims

1. An in vitro method for determining the optimal time for implantation of an embryo in a mammalian endometrium comprising detecting the levels of an endocannabinoid in the early, mid and late stage of the menstrual cycle in a subject wherein the level of the endocannabinoid in the mid stage of the cycle is higher than in each of the early and late stage and wherein a detection of a decrease of the level of the endocannabinoid in the late stage is indicative of the optimal time for embryo implantation.

2. A method as claimed in claim 1, wherein the early stage is the follicular stage, the mid stage is the ovulatory stage and the late stage is the luteal stage.

3. An in vitro method for determining the optimal time for implantation of an embryo in a mammalian endometrium comprising monitoring the levels of an endocannabinoid during the menstrual cycle of a subject and detecting the peak level of the endocannabinoid during the menstrual cycle wherein the decrease of the level of endocannabinoid following the peak is indicative of the optimal time for embryo implantation.

4. An in vitro method for detecting the entry into the luteal phase in the menstrual cycle in a subject comprising monitoring the levels of an endocannabinoid during the menstrual cycle of a subject and detecting the peak level of the endocannabinoid during the menstrual cycle wherein the decrease of the level of endocannabinoid following the peak is indicative of the entry into the luteal phase.

5. The method of any of claims 1 to 4, wherein the mammal is a human.

6. The method of any preceding claim, wherein the sample is obtainable from the subject mammal for detection.

7. The method of any preceding claim, wherein the detection is performed by means of antibodies to the endocannabinoid.

8. The method of any preceding claim, wherein the detection step comprises detection of the levels of the enzyme fatty acid amide hydrolase (FAAH) in the subject mammal.

9. An in vitro method for predicting the likelihood of a viable pregnancy in a subject mammal undergolng assisted reproductive technology comprising measuring the level of an endocannablnoldat oocyte retrieval and measuring the level of an endocannabinoid at embryo transfer, wherein a reduction in the level of said endocannabinoid from oocyte retrieval to embryo transfer indicates an increased likelihood of a viable pregnancy.

10. The method of any preceding claim, wherein the endocannabinoid is selected from the group comprising 2-arachidonoglycerol, palmitoylethanolamide and anandamide.

11. A method as claimed in claim 10, wherein the endocannabinoid is anandamide (AEA).

## Patentansprüche

1. Ein In-vitro-Verfahren zum Bestimmen des optimalen Zeitpunkts zur Implantation eines Embryos in dem Endometrium eines Säugetiers, beinhaltend das Ermitteln der Spiegel eines Endocannabinoids in der frühen, mittleren und späten Phase des Menstruationszyklus bei einem Individuum, wobei der Spiegel des Endocannabinoids in der mittleren Phase des Zyklus höher ist als in jeder von der frühen und späten Phase und wobei eine Ermittlung einer Abnahme des Spiegels des Endocannabinoids in der späten Phase den optimalen Zeitpunkt zur Embryoimplantation anzeigt.

2. Verfahren gemäß Anspruch 1, wobei die frühe Phase die Follikelphase ist, die mittlere Phase die Ovulationsphase ist und die späte Phase die Lutealphase ist.

3. Ein In-vitro-Verfahren zum Bestimmen des optimalen Zeitpunkts zur Implantation eines Embryos in dem Endometrium eines Säugetiers, beinhaltend das Überwachen der Spiegel eines Endocannabinoids während des Menstruationszyklus eines Individuums und das Ermitteln des Höchstwertspiegels des Endocannabinoids während des Menstruationszyklus, wobei die Abnahme des Spiegels von Endocannabinoid nach dem Höchstwert den optimalen Zeitpunkt zur Embryoimplantation anzeigt.

4. Ein In-vitro-Verfahren zum Ermitteln des Beginns der Lutealphase in dem Menstruationszyklus in einem Individuum, beinhaltend das Überwachen der Spiegel eines Endocannabinoids während des Menstruationszyklus eines Individuums und das Ermitteln des Höchstwertspiegels des Endocannabinoids während des Menstruationszyklus, wobei die Abnahme des Spiegels von Endocannabinoid nach dem Höchstwert den Beginn der Lutealphase anzeigt.

5. Verfahren gemäß einem der Ansprüche 1 bis 4, wobei das Säugetier ein Mensch ist.

6. Verfahren gemäß einem der vorhergehenden Ansprüche, wobei die Probe zur Ermittlung aus dem Säugetierindividuum erhalten werden kann.

7. Verfahren gemäß einem der vorhergehenden Ansprüche, wobei die Ermittlung mittels Antikörpern gegen das Endocannabinoid durchgeführt wird.

8. Verfahren gemäß einem der vorhergehenden Ansprüche, wobei der Ermittlungsschritt die Ermittlung der Spiegel des Enzyms Fettsäureamid-Hydrolase (FAAH) in dem Säugetierindividuum beinhaltet.

9. Ein In-vitro-Verfahren zum Prognostizieren der Wahrscheinlichkeit einer entwicklungsfähigen Schwangerschaft in einem Säugetierindividuum, das sich einer assistierten Reproduktionstechnik unterzieht, beinhaltend das Messen des Spiegels eines Endocannabinoids bei der Oozytengewinnung und Messen des Spiegels eines Endocannabinoids beim Embryotransfer, wobei eine Verringerung des Spiegels des Endocannabinoids von der Oozytengewinnung zu dem Embryotransfer eine erhöhte Wahrscheinlichkeit einer entwicklungsfähigen Schwangerschaft anzeigt.

10. Verfahren gemäß einem der vorhergehenden Ansprüche, wobei das Endocannabinoid aus der Gruppe ausgewählt ist, die 2-Arachidonglycerol, Palmitoylethanolamid und Anandamid beinhaltet.

11. Verfahren gemäß Anspruch 10, wobei das Endocannabinoid ein Anandamid (AEA) ist.

## Revendications

1. Une méthode in vitro pour déterminer le moment optimal pour l'implantation d'un embryon dans un endomètre de mammifère comprenant la détection des niveaux d'un endocannabinoïde au stade précoce, au stade médian et au stade tardif du cycle menstruel chez un sujet, le niveau de l'endocannabinoïde au stade médian du cycle étant plus élevé qu'au stade précoce et au stade tardif et une détection d'une baisse du niveau de l'endocannabinoïde au stade tardif étant indicative du moment optimal pour l'implantation de l'embryon.

2. Une méthode telle que revendiquée dans la revendication 1, dans laquelle le stade précoce est le stade folliculaire, le stade médian est le stade ovulatoire et le stade tardif est le stade lutéal.

3. Une méthode in vitro pour déterminer le moment optimal pour l'implantation d'un embryon dans un endomètre de mammifère comprenant la surveillance des niveaux d'un endocannabinoïde durant le cycle menstruel d'un sujet et la détection du pic de niveau de l'endocannabinoïde durant le cycle menstruel, la baisse du niveau d'endocannabinoïde après le pic étant indicative du moment optimal pour l'implantation de l'embryon.

4. Une méthode in vitro pour détecter l'entrée en phase lutéale dans le cycle menstruel chez un sujet comprenant la surveillance des niveaux d'un endocannabinoïde durant le cycle menstruel d'un sujet et la détection du pic de niveau de l'endocannabinoïde durant le cycle menstruel, la baisse du niveau d'endocannabinoïde après le pic étant indicative de l'entrée en phase lutéale.

5. La méthode de n'importe lesquelles des revendications 1 à 4, dans laquelle le mammifère est un humain.

6. La méthode de n'importe quelle revendication précédente, dans laquelle l'échantillon peut être obtenu auprès du mammifère-sujet pour la détection.

7. La méthode de n'importe quelle revendication précédente, dans laquelle la détection est réalisée au moyen d'anticorps à l'endocannabinoïde.

8. La méthode de n'importe quelle revendication précédente, dans laquelle l'étape de détection comprend la détection des niveaux de l'enzyme hydrolase des amides d'acides gras (Fatty Acid Amide Hydrolase ou FAAH) chez le mammifère-sujet.

9. Une méthode in vitro pour prédire la probabilité d'une grossesse viable chez un mammifère-sujet soumis à une technologie de reproduction assistée comprenant la mesure du niveau d'un endocannabinoïde au moment du prélèvement d'ovocytes et la mesure du niveau d'un endocannabinoïde au moment du transfert d'embryon, une réduction du niveau dudit endocannabinoïde entre le prélèvement d'ovocytes et le transfert d'embryon indiquant une probabilité accrue d'une grossesse viable.

10. La méthode de n'importe quelle revendication précédente, dans laquelle l'endocannabinoïde est sélectionné dans le groupe comprenant le 2-arachidonoglycérol, le palmitoyléthanolamide et l'anandamide.

11. Une méthode telle que revendiquée dans la revendication 10, dans laquelle l'endocannabinoïde est l'anandamide (AEA).
